# EUROPEAN PATENT APPLICATION

(11) **EP 4 309 624 A1**
(43) Date of publication of application: **24.01.2024**
(21) Application number: 21948980.4
(22) Date of filing: 20.08.2021
(51) Int. Cl.: A61F 2/24

(54) **VALVE PROSTHESIS**

(30) Priority: 07.07.2021 CN 202110767951
(71) Applicant: Shanghai Trulive Medtech Co., Ltd, Shanghai 201206 (CN); Jiangsu Trulive Medtech Co., Ltd, Rudong County Nantong Jiangsu 226400 (CN)
(72) Inventor: YANG, Wei, Shanghai 201206 (CN); WEN, Jing, Shanghai 201206 (CN)
(74) Representative: Nordmeyer, Philipp Werner
(86) International application number: PCT/CN2021/113827
(87) International publication number: WO 2023/279491

(57) **Abstract**

A valve prosthesis includes a stent (10) and a skirt assembly. The stent (10) has an inflow end (11) and an outflow end (12) opposite to the inflow end along an axial direction of the stent. The skirt assembly includes at least two skirts (20) surrounding the stent (20) in the shape of rings and each having an inner edge (21) and an outer edge (22). The inner edge (21) is an edge of the skirt (20) closer to an axis of the stent (10), and the outer edge (22) is an edge of the skirt (20) farther away from the axis of the stent (10). The inner edge (21) is attached to the stent (10), and the outer edge (22) forms a free end. The at least two skirts (20) are arranged with a spacing along the axial direction of the stent (10) and have different radial outer dimensions, and at least one of the skirts (20) is configured to be brought into contact at its outer edge (22) with native tissue (50). With this configuration, even when a small amount of blood enters a gap between the skirts (20) and the native tissue (50), it will be blocked by the skirts (20) with different radial outer dimensions, resulting in the formation of a slow blood flow region. In this region, blood can more easily coagulate, reducing paravalvular leakage and accelerating endothelialization. Moreover, the radial outer dimensions of the skirts (20) vary which roughens the skirt assembly, facilitating tissue adhesion and growth and hence endothelialization.

## Description

### TECHNICAL FIELD

The present invention relates to the field of medical devices and, in particular, to a valve prosthesis.

### BACKGROUND

The heart has four chambers: the left atrium and ventricle on the left side of the heart; and the right atrium and ventricle on the right side of the heart. It also has the ventricular inflow tracts between the atria and ventricles, the left ventricular outflow tract consisting of the left ventricle and aorta, and the right ventricular outflow tract consisting of the right ventricle and pulmonary artery. There are "one-way valves" in the ventricular inflow and outflow tracts. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent the backward flow of blood. Defects in any of these valves can induce hemodynamic changes and functional abnormalities in the heart and are called valvular heart disease.

With the development of social economy and the aging of population, the incidence of valvular heart disease has increased significantly. Studies have shown that this figure has reached up to 13.3% among those 75 years or older. Currently, traditional surgical treatment remains the first choice for patients with severe valvular disease. However, for those with advanced ages, complications in multiple organs, a history of thoracotomy or poor heart functions, the traditional surgical approach is associated with high risk and high mortality or even precludes some patients. Transcatheter valve replacement/repair has attracted extensive attention amongst academics and researchers thanks to its advantages such as no need for thoracotomy, minor trauma and rapid recovery.

However, in recent years, there are still some urgent challenges in the field of valve implantation. For example, existing valve prostheses tend to suffer from post-implantation paravalvular leakage, slow endothelialization of the surrounding tissue and shifting in position.

### SUMMARY OF THE INVENTION

It is an object of the present invention to provide a valve prosthesis, which overcomes the post-implantation problems associated with existing valve prostheses.

To this end, the present invention provides a valve prosthesis comprising a stent and a skirt assembly,
the stent having an inflow end and an outflow end opposite to the inflow end along an axial direction of the stent,
the skirt assembly comprising at least two skirts surrounding the stent in a shape of a ring and each having an inner edge and an outer edge, the inner edge being an edge of the skirt closer to an axis of the stent, the outer edge being an edge of the skirt farther away from the axis of the stent, the inner edge attached to the stent, the outer edge forming a free end,
the at least two skirts arranged with a spacing along the axial direction of the stent and having different radial outer dimensions, at least one of the skirts configured to be brought into contact at its outer edge with a native tissue.

Optionally, in a direction from the outflow end to the inflow end, the radial outer dimensions of the at least two skirts may gradually increase or decrease.

Optionally, the skirt assembly may comprise a plurality of skirts with radial outer dimensions varying at a constant rate in the direction from the outflow end to the inflow end.

Optionally, the skirt assembly may comprise a plurality of skirts with radial outer dimensions increasing or decreasing non-linearly in the direction from the outflow end to the inflow end.

Optionally, the skirt that is closer to the outflow end may overlap an adjacent skirt that is farther away from the outflow end.

Optionally, a swirling cavity is delimited by enclosing the stent and axially adjacent skirts.

Optionally, the skirt may comprise a first surface and a second surface, wherein the first surface and the second surface intersect each other to form the inner edge and are increasingly spaced apart from each other in a direction from the inner edge to the outer edge.

Optionally, the skirt may further comprise a third surface joined to each of the first surface and the second surface, wherein the first surface, the second surface and the third surface enclose to form the skirt, and wherein the outer edge is located on the third surface.

Optionally, the third surface may comprise an inwardly tapered section, wherein the inwardly tapered section extends from the outer edge to the inflow end, and is inwardly inclined toward the axis of the stent.

Optionally, the first surface, the second surface and the third surface may form a curved surface with smooth transitions.

In summary, the present invention provides a valve prosthesis including a stent and a skirt assembly. The stent has an inflow end and an outflow end opposite to each other along its axial direction. The skirt assembly includes at least two skirts surrounding the stent in the shape of rings. Each of the skirts has an inner edge closer to an axis of the stent and an outer edge farther away from the axis of the stent. The inner edge is attached to the stent, and the outer edge forms a free end. The at least two skirts are arranged with a spacing along the axial direction of the stent and have different radial outer dimensions. The outer edge of at least one of the skirts is configured to be brought into contact with native tissue.

With this configuration, during systole of the heart, the skirt assembly can block blood flow through a gap between the valve prosthesis and the tissue. Moreover, during diastole of the heart, the skirt assembly can block backflow of blood. Since the at least two skirts have different radial outer dimensions, and at least one of the skirts is configured to be brought into contact at its outer edge with the native tissue, even when a small amount of blood enters a gap between the skirts and the native tissue, it will be blocked by the skirts with different radial outer dimensions, resulting in the formation of a slow blood flow region. In this region, blood can more easily coagulate, reducing paravalvular leakage and accelerating endothelialization. Moreover, the at least two skirts with different radial outer dimensions arranged with a spacing in the axial direction roughen the skirt assembly, facilitate tissue adhesion and growth and hence endothelialization, effectively increase friction between the valve prosthesis and tissue and reduce the risk of post-implantation shifting of the valve prosthesis.

### BRIEF DESCRIPTION OF THE DRAWINGS

Those of ordinary skill in the art would appreciate that the accompanying drawings are provided to facilitate a better understanding of the present invention and do not limit the scope thereof, in which:
Fig. 1 is a schematic axial cross-sectional view of a valve prosthesis according to an embodiment of the present invention, showing skirts having radial outer dimensions gradually increasing from one after another in the direction from an outflow end to an inflow end;
Fig. 2 is an enlarged partial view of the valve prosthesis of Fig. 1;
Fig. 3 is a schematic axial cross-sectional view of a valve prosthesis according to an embodiment of the present invention, showing skirts having radial outer dimensions gradually decreasing in the direction from an outflow end to an inflow end;
Fig. 4 is a schematic axial cross-sectional view of a valve prosthesis according to an embodiment of the present invention, showing skirts having radial outer dimensions increasing or decreasing non-linearly;
Fig. 5 is a schematic axial cross-sectional view of a skirt according to an embodiment of the present invention; and
Fig. 6 is a schematic axial cross-sectional view of a valve prosthesis according to an embodiment of the present invention, showing axial cross-sections of skirts with a drop-shape.

In these figures,
10, a stent; 100, a flow passage; 11, an inflow end; 12, an outflow end; 20, a skirt; 21, an inner edge; 22, an outer edge; 23, a first surface; 24, a second surface; 25, a third surface; 250, an inwardly tapered section; 30, leaflets; 40, a swirling cavity; 41, a recess; and 50, native tissue.

### DETAILED DESCRIPTION

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, structures shown in the figures are usually a part of actual structures. In particular, as the figures tend to have distinct emphases, they are often drawn to different scales.

As used herein, the singular forms "a", "an" and "the" include plural referents. As used herein, the term "or" is generally employed in the sense of "and/or", "several" is generally employed in the sense of "at least one" and "at least two" is generally employed in the sense of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal end" generally refers to an end closer to an operator, and the term "distal end" generally refers to an end closer to a lesion in a patient. The terms "one end" and "the other end", as well as "proximal end" and "distal end", are generally used to refer to opposite portions including the opposite endpoints, rather than only to the endpoints. The terms "mounting", "coupling" and "connection" should be interpreted in a broad sense. For example, a connection may be a permanent, detachable or integral connection, a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location with respect to the other element, unless the context clearly dictates otherwise. Those of ordinary skill in the art can understand the specific meanings of the above-mentioned terms herein, depending on their context.

The inventors have found that valve prostheses have a body including a stent which is expandable either by itself or by a balloon. Since the stent usually comes in the form of a mesh-like structure, it is typically circumferentially covered with a skirt in order to prevent blood leakage from its mesh openings. However, the skirt often exhibits weak adhesion to the tissue at the implantation site. As a result, as described in the Background section, conventional valve prostheses tend to suffer from paravalvular leakage, shifting in position and other problems.

In view of the above, it is an object of the present invention to provide a valve prosthesis, which overcomes the post-implantation problems associated with existing valve prostheses. It is described below with reference to the accompanying drawings.

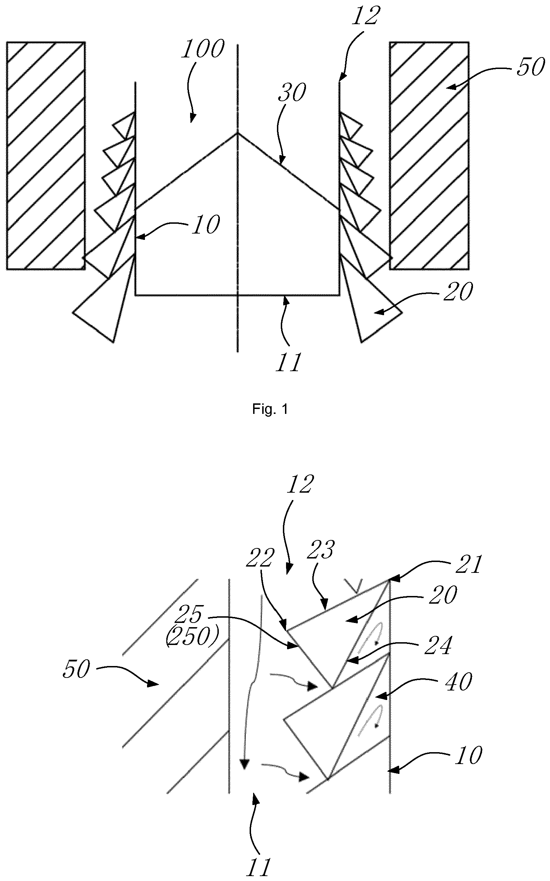

Reference is now made to Figs. 1 and 2. Fig. 1 is a schematic axial cross-sectional view of a valve prosthesis according to an embodiment of the present invention. Fig. 2 is an enlarged partial view of the valve prosthesis of Fig. 1. In an embodiment of the present invention, a valve prosthesis including a stent 10 and a skirt assembly is provided. The stent 10 has an inflow end 11 and an outflow end 12, which are opposite to each other along its axial direction (vertical as shown in Fig. 1). The skirt assembly includes at least two skirts 20 surrounding the stent 10 in the shape of rings. Each skirt 20 has an inner edge 21 and an outer edge 22. The inner edge 21 is an edge of the stent 10 closer to its axis, and the outer edge 22 is an edge of the stent 10 farther away from the axis. The inner edge 21 is attached to the stent 10, while the outer edge 22 forms a free end. The at least two skirts 20 are arranged with a spacing in the axial direction of the stent 10 and have different radial outer dimensions. At least one of the skirts 20 is configured to be brought into contact at its outer edge 22 with native tissue 50 (at an implantation site, such as a native annulus or the like). Here, it is noted that since the skirts 20 are ring-shaped, the inner edges of the ring are the inner edges 21, and their outer edges 22 are defined at the most distant points on their outer contours from the axis of the stent 10. The radial outer dimensions of the skirts 20 refer to their largest outer dimensions in the radial direction of the stent 10, i.e., the largest radial dimensions of their outer edges 22. In an alternative implementation, the skirts 20 have circular outer circumferences, and their radial outer dimensions therefore refer to their outer diameters. In case of the skirts 20 having polygonal outer circumferences, their radial outer dimensions refer to diameters of their circumcircles. The radial outer dimensions of the other components detailed below are defined in a similar way. It is noted that the present invention is not limited to any particular shapes of the outer circumferences of the stent 10 and the skirts 20, and the shapes may be circular, elliptic or polygonal, depending on the shape of the implantation site and the practical fabrication technique used.

Further, the stent 10 comprises a flow passage 100 axially extending therethrough, and the valve prosthesis further includes leaflets 30 disposed within the flow passage 100. The leaflets 30 are configured to prevent flow of a fluid from the outflow end 12 to the inflow end 11 (vertically downwards as shown in Fig. 1) through the flow passage 100. The stent 10 can provide the valve prosthesis with various functions including, among others, serving as a main segment of the valve prosthesis, carrying the internal leaflets 30 and connecting a delivery system (by providing engagement lugs or attachment lugs on the stent 10). Optionally, the stent 10 may be fabricated by braiding or cutting. Optionally, the stent 10 may be made of, for example, a nickel-titanium alloy, a titanium alloy, a cobalt-chromium alloy, MP35n, 316 stainless steel or another material. Alternatively, it may be a biocompatible metal frame or laser-cut solid metal tube made of another biocompatible metal known to those skilled in the art. Preferably, it is made of a nickel-titanium alloy. The stent 10 may also be made of an elastically or plastically deformable material. In this case, it can be used in balloon-expanded applications.

The leaflets 30 can dynamically switch between an open configuration and a closed configuration. In the closed configuration, the leaflets 30 sealingly abut against one another and are thereby tightly gathered or closed together. The leaflets 30 may be made of a suitable material or combination of materials. In some implementations, they may be selected from biological tissues, such as chemically stabilized tissues originated from heart valves of animals (e.g., pigs), or from pericardial tissues of animals such as cattle (bovine pericardia), sheep (ovine pericardia), pigs (porcine pericardia) and horses (equine pericardia), with bovine pericardial tissues being preferred. The leaflets 30 may also be made of small intestinal submucosal tissue or a synthetic material such as expanded polytetrafluoroethylene or polyester. Optionally, examples of suitable materials for the leaflets 30 may include thermoplastic polycarbonate, polyurethane, polyether-urethane, segmented polyether-urethane, silicone-polyether-urethane, silicone-polycarbonate-urethane, ultra-high molecular weight polyethylene and combinations thereof. The leaflets 30 may also be formed of a biocompatible polymer such as polyolefin, an elastomer, polyethylene glycol, polyethersulfone, polysulfone, polyvinylpyrrolidone, polyvinyl chloride, a fluoropolymer, a silicone polyester, a silicone polymer and/or oligomer, and/or polylactone or a block copolymer thereof, or a combination thereof. Optionally, the leaflets 130 may have been subjected to a surface treatment using an anticoagulant (or undergone a reaction with anticoagulant). Examples of the anticoagulant may include, but are not limited to, heparinized polymers. Those skilled in the art may appropriately configure the materials, structures and dimensions of the leaflets 30 and the stent 10 according to currently available techniques, such as the interventional prosthetic heart valve disclosed in Chinese Patent Publication No. CN105726167A and the heart valve prosthesis disclosed in Chinese Patent Publication No. CN104000672A, the entire contents of which are hereby incorporated by reference herein. The valve prosthesis according to the present embodiment may be deployed at an aortic valve, a pulmonary valve, a mitral valve or a tricuspid valve, and those skilled in the art may appropriately configure the shapes, dimensions, structures, materials and number of skirts 20 in the skirt assembly depending on the deployment site.

Referring to Figs. 1 and 2, preferably, each skirt 20 extends from the inner edge 21 to the outer edge 22 so as to be flared toward the inflow end 11. During systole of the heart, blood flows in the direction from the inflow end 11 to the outflow end 12 (upwards as shown in Fig. 1). At this time, since the annular outer edges 22 of the skirts 20 are oriented outwardly, and at least one of the skirts 20 remains in contact at its outer edge 22 with the native tissue 50, the skirt assembly can block blood from flowing through a gap between the valve prosthesis and the native tissue 50. Referring to Fig. 2, during diastole of the heart, blood flows in the opposite direction from the outflow end 12 to the inflow end 11 (downwards as shown in Fig. 2). As the leaflets 30 are in the closed configuration, backflow of blood through the interior of the stent 10 is prevented. Moreover, since the at least one of the at least two skirts 20 with different radial outer dimensions is configured to be brought into contact at its outer edge with the native tissue 50, even when a small amount of blood enters a gap between the skirts 20 in the skirt assembly and the native tissue 50, it will be blocked by the skirts 20 with different radial outer dimensions, resulting in the formation of a slow blood flow region. As a result, coagulation of blood will occur overtime, which can accelerate endothelialization. Therefore, the skirt assembly can not only block backflow of blood, but also allows blood that regurgitates a gap between the valve prosthesis and the native tissue 50 to be coagulated. In addition, the at least two skirts 20 with different radial outer dimensions and axially arranged with a spacing impart greater roughness to the skirt assembly, which helps in adhesion and growth of the native tissue 50 thereon and facilitates endothelialization. Further, friction between the valve prosthesis and the native tissue 50 can be effectively increased, lowering the risk of post-implantation shifting of the valve prosthesis.

Optionally, the inner edges 21 of the skirts 20 are fixedly attached to the stent 10 by gluing or suturing. Preferably, the inner edges 21 are attached to the stent 10 by suturing. Additionally, referring to Fig. 2, the outer edge 22 of skirt 20 closer to outflow end 12 overlaps an adjacent skirt that is located farther away from the outflow end 12. Here, by "overlaps", it is intended to mean that, out of the adjacent skirts 20, the skirt 20 closer to the outflow end 12 abuts against the skirt 20 farther away from the outflow end 12. In particular, here, by "abuts against", it is intended to mean that the two are not fixedly connected, with a gap allowing passage of a small amount of blood therethrough being present where the abutment occurs. Further, the axially adjacent skirts 20 and the stent 10 enclose to form irregularly shaped swirling cavity(ies) 40 in communication with said gap(s). When a small amount of blood passes through the gap(s) into the swirling cavity(ies) 40, a slow blood flow region will be formed, in which blood can more easily coagulate, reducing paravalvular leakage and accelerating endothelialization.

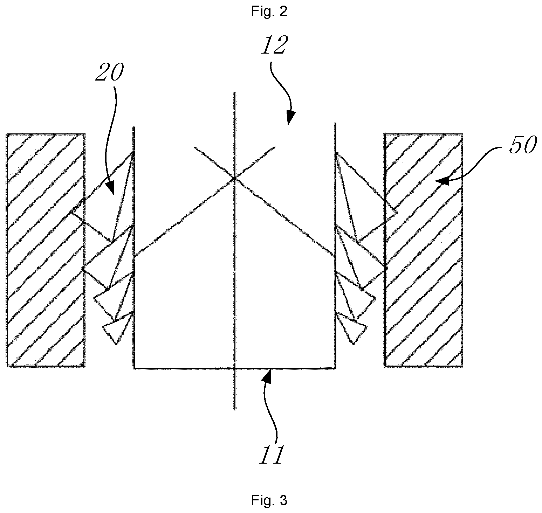

Referring to Figs. 1 and 3, in some implementations, the radial outer dimensions of the at least two skirts 20 increase or decrease in the direction from the outflow end 12 to the inflow end 11. Compared with conventional valve prostheses (having a skirt with a constant radial dimension), this configuration allows the valve prosthesis to have a reduced overall crimped size, which can reduce damage caused to the human body during the implantation process. In case of the radial outer dimensions of the multiple skirts 20 increasing in the direction from the outflow end 12 to the inflow end 11 (see Fig. 1), during systole of the heart, the larger skirt(s) 20 can block the blood flowing through the gap between the native tissue 50 and the valve prosthesis. Moreover, during diastole of the heart, the blood that regurgitate flows between the multiple skirts 20, forming a slow flow region helping in reducing paravalvular leakage. In case of the radial outer dimensions of the multiple skirts 20 decreasing in the direction from the outflow end 12 to the inflow end 11 (see Fig. 3), during systole of the heart, blood pumped from the ventricles flows between the multiple skirts 20 and enters the swirling cavities 40, forming a slow blood flow region that accelerates endothelialization. During diastole of the heart, the larger skirt(s) 20 can block backflow of blood, helping in reducing paravalvular leakage.

Further, in the skirt assembly, the radial outer dimensions of the multiple skirts 20 may vary at a constant rate in the direction from the outflow end 12 to the inflow end 11. Here, by "the radial outer dimensions of the multiple skirts 20 may vary at a constant rate", it is intended to mean that the radial outer dimensions of the multiple skirts 20 may vary linearly so that the skirt assembly is generally in the shape of a truncated cone. The linear variation enables the valve prosthesis to be uniformly stressed when it is being released. Moreover, after the valve prosthesis is implanted, the speed of regurgitation blood will vary at a constant rate over the skirts 20. This is helpful in endothelialization. Of course, in other implementations, the radial outer dimensions of the multiple skirts 20 may vary at a non-constant rate so that, for example, the skirt assembly overall comprises a flared shape.

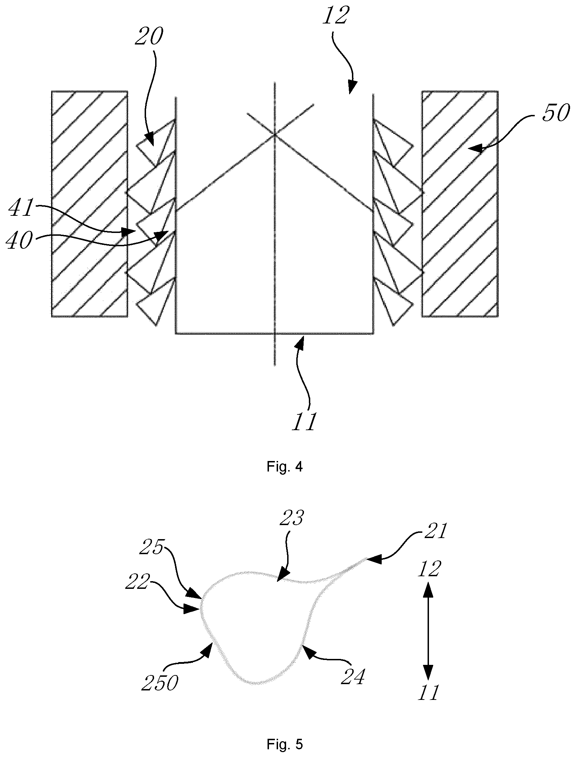

Referring to Fig. 4, in some other implementations, the radial outer dimensions of the multiple skirts 20 in the skirt assembly may increase or decrease non-linearly in the direction from the outflow end 12 to the inflow end 11. Here, by "increase or decrease non-linearly", it is intended to mean that, out of the multiple skirts 20 arranged in the axial direction of the stent 10, there is at least one middle skirt 20 whose radial outer dimension is greater or smaller than the radial outer dimensions of the skirts located two ends along the axis. It would be appreciated that this requires the skirt assembly to have at least three skirts 20. For example, in case of the skirt assembly including three skirts 20, the radial outer dimension of the middle skirt 20 may be greater or smaller than the radial outer dimensions of the skirts located two ends along the axis. Thus, the skirt assembly will have one raised or recessed portion and thus be generally gourd- or drum-shaped. When the skirt assembly includes more skirts 20, it is not limited to having only one raised or recessed portion, and each raised or recessed portion is not limited to being formed by only one skirt 20. For example, the skirt assembly may be generally wavy in shape. Additionally, it is noted that, in some examples, two skirts 20 adjacent to each other in the axial direction of the stent 10 may have the same radial outer dimension. For example, the skirt assembly may have four skirts 20, in which the middle two skirts 20 have the same radial outer dimension that is smaller or greater than the radial outer dimensions of the two skirts 20 located at two ends along the axis. Preferably, in case of the radial dimensions of the multiple skirts 20 increasing or decreasing non-linearly, at least two larger skirts 20 are brought into contact with the native tissue 50. This can result in an increased contact area between the valve prosthesis and the native tissue 50, enhance anchoring of the valve prosthesis, reduce the risk of shifting of the valve prosthesis, facilitate adhesion and growth of tissue thereon and accelerate endothelialization. Further, a recess 41 may be formed between adjacent larger skirts 20, which can act in synergy with the swirling cavity 40 formed by the skirts 20 to more effectively slow down blood flow and introduce turbulence therein, thus facilitating coagulation.

Preferably, each skirt 20 includes a first surface 23 and a second surface 24, which intersect each other at the inner edge 21 and are increasingly spaced from each other in the direction from the inner edge 21 to the outer edge 22. Both the first surface 23 and the second surface 24 extend outwardly from the inner edge 21 and are increasingly spaced from each other. That is, each skirt 20 has a thickness gradually increasing from the inner edge 21 outwards. Such skirts 20 thinner at positions closer to the stent 10 and thicker at positions farther away from the stent 10 comprises a series of ridges across the outer circumference of the valve prosthesis, which roughens the outer circumference and facilitates adhesion and growth of the native tissue 50 thereof and hence endothelialization. Further, friction between the valve prosthesis and the native tissue 50 can be effectively increased, lowering the risk of post-implantation shifting of the valve prosthesis. It is noted that the first surface 23 and the second surface 24 are not limited to being increasingly spaced from each other linearly at a constant rate and may be increasingly spaced from each other at a varying rate. That is, the first surface 23 and the second surface 24 are not limited to being flat surfaces. At least one of them may be curved, corrugated or otherwise non-flat.

Referring to Figs. 2 and 5, each skirt 20 may further include a third surface 25 intersecting both the first surface 23 and the second surface 24. The first surface 23, the second surface 24 and the third surface 25 enclose to form the skirt 20, and the outer edge 22 is on the third surface 25. The third surface 25 is contiguous with the first surface 23 and the second surface 24 at their ends farther away from the inner edge 21. Thus, it together with the first surface 23 and the second surface 24 forms a closed loop and forms the skirt 20. The present invention is not limited to any particular shape of the third surface 25, and the surface may either be flat as shown in Fig. 2 or curved as shown in Fig. 5. It would be appreciated that the outer edge 22 may be located at the intersection of the third surface 25 with either the first surface 23 or the second surface 24, or at the middle portion of the third surface 25.

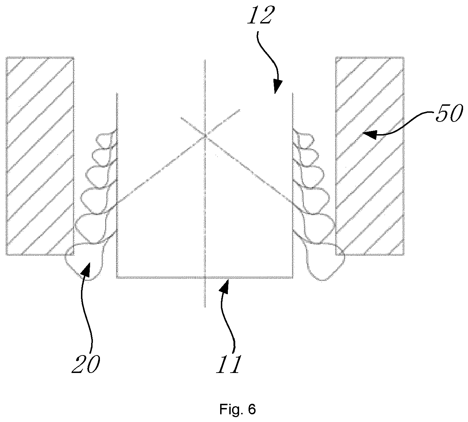

Preferably, the third surface 25 includes an inwardly tapered section 250 extending from the outer edge 22 toward the inflow end 11 and inwardly inclined toward the axis of the stent 10. In the example shown in Fig. 2, the third surface 25 is flat and entirely serves as the inwardly tapered section 250, and the outer edge 22 is located at the intersection of the third surface 25 and the first surface 23. The third surface 25 is inwardly inclined toward the axis of the stent 10 in the direction from the outflow end 12 to the inflow end 11. In the example shown in Fig. 6, the third surface 25 is curved, and the outer edge 22 is located at middle portion of the third surface 25. That is, the third surface 25 is convex outwardly. In this case, the inwardly tapered section 250 is a part of the third surface 25. When blood flows over an outer edge of the inwardly tapered section 250, it becomes turbulent and slowed down, facilitating coagulation.

Referring to Figs. 1 and 2, in an alternative exemplary implementation, each skirt 20 has a triangular cross-section taken along a radial direction of the ring that it forms. The inner edge 21 is located at one corner of the triangle, and the outer edge 22 is on the side of the triangle opposite to said corner. Since cross-section of skirts 20 comprises a triangle, the skirts 20 can be fabricated easily, and the obtained skirt assembly can work in a desirable way. In this case, the swirling cavity(ies) 40 delimited by enclosing the skirts 20 and the stent 10 also has/have substantially triangular cross-section(s), which can effectively decelerate blood flow and add turbulence thereto, thus facilitating coagulation. It would be appreciated that the terms "triangle" and "triangular" are to be interpreted in a broader sense to include any shapes substantially having three sides, such as rounded triangles, curved triangles, etc.

Referring to Figs. 5 and 6, in an alternatively example, the first surface 23, the second surface 24 and the third surface 25 form a curved surface with smooth transitions so that the skirt 20 has a drop-shaped cross-section taken along a radial direction of the ring that it forms. With this configuration, apart from reduced paravalvular leakage and accelerated endothelialization, since the skirts 20 have rounded and smooth edges, when they come into contact with tissue, less damage will be caused thereto.

Optionally, materials suitable for fabrication of the skirts 20 may include knitted, woven and braided fabric. Preferably, they are made of polyester fabric. Preferred materials for the skirts 20 may include PTFE and/or ePTFE.

In summary, the present invention provides a valve prosthesis including a stent and a skirt assembly. The stent has an inflow end and an outflow end opposite to the inflow end along its axial direction. The skirt assembly includes at least two skirts surrounding the stent in the shape of rings. Each of the skirts has an inner edge closer to an axis of the stent and an outer edge farther away from the axis of the stent. The inner edge is attached to the stent, and the outer edge forms a free end. The at least two skirts are arranged with a spacing along the axial direction of the stent and have different radial outer dimensions. The outer edge of at least one of the skirts is configured to be brought into contact with native tissue. With this configuration, during systole of the heart, the skirt assembly can block blood flowing through gaps between the valve prosthesis and the tissue. Moreover, during diastole of the heart, the skirt assembly can block backflow of blood. Since the at least two skirts have different radial outer dimensions, and at least one of the skirts is configured to be brought into contact at its outer edge with the native tissue, even when a small amount of blood enters between the skirts and the native tissue, it will be blocked by the skirts with different radial outer dimensions, resulting in the formation of a slow blood flow region. In this region, blood can more easily coagulate, reducing paravalvular leakage and accelerating endothelialization. Moreover, the at least two skirts with different radial outer dimensions axially arranged with a spacing roughen the skirt assembly, facilitate tissue adhesion and growth and hence endothelialization, effectively increase friction between the valve prosthesis and tissue and reduce the risk of post-implantation shifting of the valve prosthesis.

It is noted that the foregoing several embodiments may be combined. The description presented above is merely that of some preferred embodiments of the present invention and is not intended to limit the scope thereof. Any and all changes and modifications made by those of ordinary skill in the art based on the above teachings fall within the scope as defined in the appended claims.

## Claims

1. A valve prosthesis, comprising a stent and a skirt assembly,
wherein the stent comprises an inflow end and an outflow end opposite to the inflow end along an axial direction of the stent,
wherein the skirt assembly comprises at least two skirts each surrounding the stent in a shape of a ring and having an inner edge and an outer edge, wherein the inner edge is an edge of the skirt closer to an axis of the stent, wherein the outer edge is an edge of the skirt farther away from the axis of the stent, and wherein the inner edge is attached to the stent, and the outer edge forms a free end,
wherein the at least two skirts are arranged with a spacing along the axial direction of the stent and have different radial outer dimensions, and wherein the outer edge of at least one of the skirts is configured to be brought into contact with a native tissue.

2. The valve prosthesis according to claim 1, wherein in a direction from the outflow end to the inflow end, the radial outer dimensions of the at least two skirts gradually increase or decrease.

3. The valve prosthesis according to claim 2, wherein the skirt assembly comprises a plurality of skirts with radial outer dimensions varying at a constant rate in the direction from the outflow end to the inflow end.

4. The valve prosthesis according to claim 1, wherein the skirt assembly comprises a plurality of skirts with radial outer dimensions increasing or decreasing non-linearly in the direction from the outflow end to the inflow end.

5. The valve prosthesis according to claim 1, wherein the skirt that is closer to the outflow end overlaps an adjacent skirt that is farther away from the outflow end.

6. The valve prosthesis according to claim 5, wherein a swirling cavity is delimited by enclosing the stent and axially adjacent skirts.

7. The valve prosthesis according to claim 1, wherein the skirt comprises a first surface and a second surface, and wherein the first surface and the second surface intersect each other to form the inner edge and are increasingly spaced apart from each other in a direction from the inner edge to the outer edge.

8. The valve prosthesis according to claim 7, wherein the skirt further comprises a third surface joined to each of the first surface and the second surface, wherein the first surface, the second surface and the third surface enclose to form the skirt, and wherein the outer edge is located on the third surface.

9. The valve prosthesis according to claim 8, wherein the third surface comprises an inwardly tapered section, wherein the inwardly tapered section extends from the outer edge to the inflow end, and is inwardly inclined toward the axis of the stent.

10. The valve prosthesis according to claim 8, wherein the first surface, the second surface and the third surface form a curved surface with smooth transitions.
